# EUROPÄISCHE PATENTSCHRIFT

(11) **EP 2 884 987 B1**
(45) Veröffentlichungstag und Bekanntmachung des Hinweises auf die Patenterteilung: **27.02.2019**
(21) Anmeldenummer: 13748310.3
(22) Anmeldetag: 13.08.2013
(51) Int. Cl.: A61K 36/15, A61K 36/185

(54) **TIERFUTTER- ODER TRÄNKWASSERZUSATZ FÜR WIEDERKÄUER**
ADDITIVE FOR ANIMAL FOOD OR DRINKING WATER FOR RUMINANTS
ADDITIF ALIMENTAIRE OU À L'EAU POTABLE POUR ANIMAUX RUMINANTS

(30) Priorität: 14.08.2012 AT 8882012; 20.08.2012 AT 9042012
(43) Veröffentlichungstag der Anmeldung: 24.06.2015
(73) Patentinhaber: Neufeld, Klaus, 2532 Heiligenkreuz (AT)
(72) Erfinder: Neufeld, Klaus, 2532 Heiligenkreuz (AT)
(74) Vertreter: Puchberger & Partner Patentanwälte
(86) Internationale Anmeldenummer: PCT/EP2013/066872
(87) Internationale Veröffentlichungsnummer: WO 2014/026965

(56) Entgegenhaltungen:
- EP-A1- 2 085 083
- WO-A1-2011/127499
- WO-A1-2012/002871
- DATABASE WPI Week 200967 Thomson Scientific, London, GB; AN 2009-L64723 XP002713124, -& CN 101 474 344 A (LANZHOU INST ANIMAL SCI&VETERINARY PHA) 8. Juli 2009 (2009-07-08)
- LEIBETSEDER J ET AL: "Crude fiber dietary fiber", KRAFTFUTTER - FEED MAGAZINE, FRANKFURT, DE, Bd. 89, Nr. 10, 1. Januar 2006 (2006-01-01), Seiten 24-28, XP008089625, ISSN: 0023-4427

## Beschreibung

### Gebiet der Erfindung

Die Erfindung betrifft Zusätze zum Tierfutter oder Tränkwasser für Wiederkäuer.

### Hintergrund der Erfindung

Die landwirtschaftliche tierische Produktion zielt heute darauf ab, unter möglichst ökonomischen Bedingungen ein akzeptables Leistungsniveau der Nutztiere und einen hohen Gesundheitsstatus zu erreichen. Es hat sich gezeigt, dass ein guter Gesundheitszustand in der Folge auch zu guten Leistungen in Hinblick auf Wachstum, Milchleistung, Wollproduktion, Reproduktion etc. führt. In früherer Zeit hat man versucht, das Leistungsniveau durch sogenannte nutritive Gaben von antimikrobiellen Wirkstoffen (z.B. Antibiotika) zu erhöhen. Im Bereich der Wiederkäuer wurde lange Zeit beispielsweise das Produkt Flavomycin® (Substanzname: Flavophospholipol) verwendet [Van der Merwe BJ et al: The effect of flavophospholipol (Flavomycin®) on milk production and milk urea nitrogen concentrations of grazing dairy cows, South African Journal of Animal Science (SASAS) 2001, 31(2): 101-105]. Ein weiteres Beispiel für die Gruppe dieser Substanzen sind die ionophoren Antibiotika, beispielsweise das Produkt Rumensin (Substanzname: Monensin-Natrium) [Callaway TR et al: lonophores: Their use as ruminant growth promotants and impact on food safety, Curr. Issues Intest. Microbiol. 2003, 4: 43-51], das jedoch mittlerweile in der EU als Futtermittelzusatz für Wiederkäuer verboten ist. Monensin wird von *Streptomyces cinnamonensis* gebildet. Im Rahmen der weitverbreiteten früheren Anwendung von Monensin beim Wiederkäuer konnte nachgewiesen werden, dass Monensin über einen Einfluss auf die Aktivität der Pansenflora auch einen positiven Effekt auf die Prävalenz der Pansenazidose hat. Dieser Einfluss ist dadurch gegeben, dass Monensin eine überschießende Kohlenhydratverwertung und damit Übersäuerung des Pansens verhindert, und zwar durch einen Einfluss auf die Aktivität der Pansenflora ("dämpfender Effekt" auf die mikrobielle Flora). Diese Wirkung konnte auch mit anderen antimikrobiellen Wirkstoffen nachgewiesen werden. Ein unmittelbarer Einfluss auf kohlenhydratspaltende Enzyme ist durch antimikrobielle Wirkstoffe nicht gegeben.

Der Einsatz der antimikrobiellen Wirkstoffe ist mit bedeutenden Nachteilen verbunden. Einerseits wurde aus wissenschaftlicher Sicht nicht immer ein positiver Effekt dieser Substanzen auf das Leistungsniveau der Nutztiere nachgewiesen [Gritzer K und Leitgeb R: Überprüfung der Wirksamkeit antibiotischer und mikrobieller Leistungsförderer in der Rindermast, Die Bodenkultur, 1998, 49(1): 51-59], andererseits aber hat sich im Bereich des Konsumenten ein Trend zu natürlichen und rückstandsfreien tierischen Nahrungsmitteln entwickelt und somit im Handel und beim Konsumenten ein Widerstand gegen die Verwendung solcher Substanzen in der Landwirtschaft aufgebaut. Außerdem hat sich auf wissenschaftlicher Ebene der Verdacht erhärtet, dass es durch die Verwendung antibiotischer Leistungsförderer in der Landwirtschaft zu einer Entwicklung von Resistenzen gegen Antibiotika, insbesondere auch bei der Verwendung in der Humanmedizin, kommen kann. Als alternative Produkte wurden in den letzten Jahren sogenannte Lebendhefen eingesetzt. Ihre fördernde Wirkung auf Bakterien im Pansen sowie ihre pH-stabilisierende Wirkung im Pansen durch eine bessere Milchsäureverwertung sind bekannt [Schmitz W: Lebendhefen: Kleine Zellen mit großer Wirkung, Der fortschrittliche Landwirt 2010, 20: 10-12].

Auf veterinärmedizinischer Ebene hat sich jedoch herausgestellt, dass die größte Kapazität für eine optimale Leistung der landwirtschaftlichen Nutztiere der Erhalt eines optimalen Gesundheitszustandes ist. Bei den wiederkäuenden landwirtschaftlichen Nutztieren, wie beispielsweise Kühen, Schafen oder Ziegen, gibt es zwei große Stoffwechselprobleme, die zu erheblichen Leistungseinbußen führen. Es handelt sich dabei einerseits um die Ketose, andererseits um die Azidose, insbesondere die subakute Azidose (SARA). Ketose und Azidose sind in ihrer wirtschaftlichen Bedeutung ungefähr gleichwertig.

Die subakute Rumenazidose (SARA) tritt je nach untersuchtem Land und Haltungsform in einer Häufigkeit von 10% bis 30% der Milchkühe auf. So geben etwa Enemark und Jorgensen (2001) bei Milchkühen in Dänemark eine Häufigkeit von 22% an, Oetzel (2003) gibt die Häufigkeit von subklinischer Pansenazidose bei frischlaktierenden Kühen mit 15% an. Somit stellt die Azidose neben der Ketose das größte und aus ökonomischer Sicht wichtigste Gesundheitsproblem in der Rinderproduktion dar.

### Stand der Technik

Bisherige Methoden, Pansenazidose zu verhindern, sind von begrenzter Wirksamkeit. Üblicherweise werden dem Tierfutter Puffersubstanzen, insbesondere Natriumbicarbonat, zugesetzt. Allerdings führt Natriumbicarbonat in wirksamen Dosierungen von >150 g pro Tier und Tag zu einer verminderten Futteraufnahme und kann daher nur begrenzt eingesetzt werden.

Aus EP 1 157 696 ist die Verwendung von Acarbose und Trestatinen zur Herstellung eines Medikaments für die kurative, palliative und prophylaktische Behandlung von Pansenazidose bekannt.

Aus der WO 2011/127499 ist ein Tierfutterzusatz bekannt, der ein Lignocellulosehältiges, Eisenionen-bindendes Material, wie Holz, Holzfasern, Rinde oder Rindenfasern von Pinusarten, in Kombination mit einem pflanzlichen, antimikrobiellen Wirkstoff, wie einem Extrakt oder Pflanzenmaterial von Pflanzen der Gattung Magnolia oder der Gattung der Cinnamomumgewächse, enthält. Die Wirkung der antimikrobiellen Wirkstoffe, z.B. Magnolol und Honokiol, wird durch die Kombination mit der Eisenionenbindenden Komponente optimiert, da die Eisenionen-bindende Komponente die Verfügbarkeit des Eisens verringert, wodurch Bakterien der Pansenflora, die zum Wachstum Eisen benötigen, sensitiver für die Wirkung der antimikrobiellen Wirkstoffe werden.

Aus EP 2 085 083 ist die Verwendung einer Mischung von Naringin, Bitterorangenextrakt und Sepiolith zur Verbesserung der Pansenfermentation bekannt.

Aus CN 101 474 344 A ist ein getrocknete Orangenschale, Rhizoma Atractylodis, Buerger Pipewort, Alaun, Chinesischer-Lebensbaum-Zweig, Ulmenrinde, Knoblauch, Cyrtomium-Wurzel, Folium Cortex Eucommiae und Fenchel enthaltendes Tierfutter für Schafe zur Verbesserung der Verdauung bekannt.

Aus der WO 2012/002871 ist eine Johannisbrot, ein Silicatmineral und ein Glukan pflanzlichen Ursprungs enthaltende Zusammensetzung zur Bindung und Inaktivierung von Mykotoxinen in einem Tierfutter bekannt.

Aus Leibetseder et al., "Crude fiber dietary fiber", Kraftfutter- Feed Magazine, Frankfurt, Bd. 89, Nr. 10, 1. Jan.. 2006, Seiten 24-28, ist die Verwendung von Mykotoxin-freien Rohfaserprodukten aus Lignocellulose in der Nutztierernährung bekannt.

### Offenbarung der Erfindung

Die vorliegende Erfindung hat sich die Aufgabe gestellt, Mittel zur Behandlung oder Prophylaxe von akuter oder subakuter Pansenazidose oder aus Pansenazidose resultierenden Zuständen bei Wiederkäuern bereitzustellen. Diese Mittel umfassen ein Tierfutter, einen Tierfutterzusatz bzw. eine Vormischung zur Herstellung eines Tierfutters sowie einen Tränkwasserzusatz.

Gemäß einem Aspekt gibt die vorliegende Erfindung einen Tierfutterzusatz zur Behandlung oder Prophylaxe von akuter oder subakuter Pansenazidose oder aus Pansenazidose resultierenden Zuständen bei Wiederkäuern an, der mit Eisenionen und/oder Eisenverbindungen komplexiert vorliegende oligomere Procyanidine enthält, wobei das Verhältnis zwischen Eisenionen und/oder Eisenverbindungen und oligomeren Procyanidinen zwischen 1:10 und 1:20 000, vorzugsweise zwischen 1:100 und 1:8 000 liegt und wobei die oligomeren Procyanidine in dem Tierfutterzusatz in Form von vermahlener Kiefernrinde, Kiefernrindenextrakt oder einer beliebigen Mischung davon enthalten sind. Im Rahmen der vorliegenden Erfindung hat sich überraschenderweise gezeigt, dass die Rinde von Kiefernarten (Pinus spp.) oder Ulmen (Ulmus spp., nicht gemäß der Erfindung) oder Extrakte solcher Rinden eine positive, auch vorbeugende, Wirkung auf das Azidosegeschehen haben. Dies erfolgt physiologisch insbesondere durch die in den genannten Rinden enthaltenen oligomeren Procyanidine (die auch als Proanthocyanidine bezeichnet werden). Für diese Substanzen konnte eine Hemmwirkung auf die Pansenamylase im Rahmen von *In-vitro-Tests* mit Pansensaft nachgewiesen werden.

Beispielsweise kann die Herstellung des erfindungsgemäßen Tierfutterzusatzes, in dem die oligomeren Procyanidine als eisenhaltiger Komplex vorliegen, folgendermaßen erfolgen: Unter Verwendung einer kommerziell verfügbaren Eisenchlorid- oder Eisenlaktatlösung oder dergleichen wird vermahlene Kiefernrinde dem gewünschten Verhältnis an Eisenionen und Eisenverbindungen zu oligomeren Procyanidinen entsprechend besprüht und anschließend schonend getrocknet.

Eine weitere Ausführungsform ist die Vermischung von Procyanidin-haltigen Extrakten beispielsweise mit den genannten Eisenlösungen im gewünschten Verhältnis. Es können hier auch wasserlösliche Procyanidin-haltige Extrakte verwendet werden, die dann unter Beigabe der Eisenlösungen die erfindungsgemäßen Komplexe bilden. Diese Ausführungsform kann insbesondere auch in flüssiger Form weiter verarbeitet oder eingesetzt werden, d.h. in flüssiger Form auf Trägerstoffe, Futtermittelvormischungen etc. aufgesprüht werden, den Tieren aber auch in flüssiger Form beispielsweise mittels Drench eingegeben werden.

Für die mit Eisenionen und/oder Eisenverbindungen komplexiert vorliegenden oligomeren Procyanidine konnte eine deutliche Verbesserung der Hemmwirkung auf die Pansenamylase *in vitro* nachgewiesen werden.

Aus physiologischer Sicht ist es wichtig, dass bei Tieren, die keine Pansenazidose entwickeln oder für diese nicht empfänglich sind, keine Hemmung der Pansenamylase stattfindet, da eine solche Hemmung bei "gesunden" Tieren zu einer Entgleisung der Pansenfermentation und in der Folge zu gesundheitlichen Störungen und zu einer Leistungsdepression führen kann. Von den beim Stand der Technik verwendeten Hemmstoffen der Pansenamylase (z.B. Acarbose) ist bekannt, dass sie die Pansenamylase unabhängig vom Gesundheitszustand der Tiere hemmen.

Im Rahmen der Erfindung wurden umfangreiche Untersuchungen durchgeführt, wobei überraschenderweise festgestellt wurde, dass Kiefernrinde, Ulmenrinde (nicht gemäß der Erfindung) oder Extrakte davon einen Wirkungsmechanismus zeigen, der durch einen Abfall des pH-Wertes (d.h. einen Säureanstieg) getriggert (d.h. ausgelöst) wird. Die in der Kiefern- und Ulmenrinde enthaltenen oligomeren Procyanidine werden durch ein Abfallen des pH-Wertes in den für die Entstehung der Pansenazidose oder der subakuten Pansenazidose sensiblen Bereich freigesetzt und damit aktiv. Die freigesetzte Menge und die Freisetzungsgeschwindigkeit können durch den Extraktionsgrad der Rinden beeinflusst werden. Die vermahlene, d.h. nicht extrahierte, Rinde zeigt die größte Verzögerung der Wirkstofffreisetzung und hat sich daher in der Praxis am besten bewährt, um gezielt bei azidosesensiblen Tieren über die Hemmung der Pansenamylase einen pH-Abfall zu vermeiden, ohne das Risiko einzugehen, bei gesunden oder nicht sensiblen Tieren die Pansenphysiologie unnötig zu stören.

Wie sie in dieser Beschreibung verwendet werden, sind die Ausdrücke "akute Pansenazidose" und "subakute Pansenazidose" wie folgt zu verstehen. Die akute Pansenazidose entsteht durch eine überschießende Fermentationsaktivität im Pansen mit daraus resultierender Bildung von Säuren als Stoffwechselprodukte des Fermentationsprozesses. Grund hierfür ist oftmals eine hohe Kraftfuttergabe, die beim Einsetzen der Laktation notwendig ist, um das entstehende Energiedefizit durch die Milchbildung zu begrenzen. Ein lange andauerndes Absinken des pH-Wertes im Pansen unter einen Wert von 5,5 bedingt eine Veränderung der Pansenflora, Sistieren der Wiederkautätigkeit, Inappetenz, Durchfall, Koliken bis zu schweren Störungen des Allgemeinbefindens und Festliegen. Der niedrige pH-Wert schädigt die Schleimhaut des Pansens, die dann ihre Barrierefunktion nicht mehr erfüllen kann. Der Übertritt von Bakterien, biogenen Aminen, Pilzen, Pilz- und Bakterientoxinen aus dem Pansen in den Organismus wird begünstigt und verschlimmert das Krankheitsbild.

SARA (subakute Rumenazidose) stellt ein weit verbreitetes Problem in der Rinderproduktion dar. SARA ist mit einem Abfall des Pansen-pH-Wertes unter die physiologische Norm assoziiert. Die Ursache von Pansenazidose ist in der Regel ein Überangebot an rasch fermentierbaren Kohlenhydraten, oft in Verbindung mit einem Mangel an strukturierter Rohfaser. Im Gegensatz zur akuten Pansenazidose sind die Phasen eines unphysiologisch niedrigen pH-Werts (unter 5,8) bei SARA zeitlich begrenzt und dauern von wenigen Minuten bis zu mehreren Stunden. Halten diese azidotischen Phasen länger als drei bis vier Stunden an, so kommt es zu einer morphologischen Schädigung der Pansenschleimhaut, die in der Regel *erst post mortem* feststellbar ist. Diese Schädigung bedingt wiederum eine erhöhte Durchlässigkeit für Bakterien, biogene Amine, Pilze, Pilz- und Bakterientoxine, welche im Organismus Entzündungen hervorrufen und Leberschäden sowie Klauenerkrankungen begünstigen oder gegebenenfalls sogar in die Milch ausgeschieden werden können. Als Folgeerkrankungen treten auf: Klauenerkrankungen (Laminitis), Störungen der Lebergesundheit (insbesondere Abszesse), Fruchtbarkeitsstörungen, verminderte Milchleistung sowie verminderter Fleischansatz bei Mastrindern. Weiters kommt es durch die Störung der Physiologie der Pansenzotten zu Fressunlust (Inappetenz) und einer Veränderung der Kotkonsistenz (d.h. der Kot wird dünnbreiig bis wässrig).

Beispiele für aus Pansenazidose resultierende Zustände bei einem Wiederkäuer, die durch Zusetzen von vermahlener Kiefernrinde, Kiefernrindenextrakt oder einer beliebigen Mischung davon, wie oben beschrieben mit eisenhaltiger Lösung behandelt, zum Tierfutter oder Tränkwasser behandelt, reduziert oder vermieden werden können, sind pH-Absenkung im Pansen, Entgleisung der Pansenflora, Sistieren der Wiederkautätigkeit, Abfall der Milchmenge, Abfall des Milchfettgehaltes, chronische Stoffwechselbelastung, Belastung des Leberstoffwechsels, Unfruchtbarkeit, verlängerte Remontierungsrate, geringere Kälberzahl und entzündliche Klauenerkrankungen.

Gemäß einem weiteren Aspekt der vorliegenden Erfindung führt die Verwendung des erfindungsgemäßen Futterzusatzes auch zu einer reduzierten Aflatoxinausscheidung in die Milch der Wiederkäuer. Diese Reduktion der Aflatoxinausscheidung steht mit der durch die Verwendung des erfindungsgemäßen Futterzusatzes deutlich reduzierten Azidosefrequenz in Zusammenhang. Es wird zum einen davon ausgegangen, dass durch die reduzierte Azidosefrequenz weniger Schleimhautschäden im Pansen vorhanden sind, sodass die Schleimhaut ihre Barrierefunktion besser erfüllen kann. Zum anderen bewirkt der erfindungsgemäße Futterzusatz generell eine bessere Pansenaktivität, wobei es auch zu einem Abbau von Aflatoxinen kommt.

Gemäß einem anderen Aspekt betrifft die Erfindung ein Tierfutter für Wiederkäuer, das den wie vorstehend beschrieben mit Eisenionen und/oder Eisenverbindungen komplexierte oligomere Procyanidine enthaltenden Tierfutterzusatz in einer Menge, die zur Behandlung oder Prophylaxe von akuter oder subakuter Pansenazidose oder aus Pansenazidose resultierenden Zuständen bei einem Wiederkäuer wirksam ist, sowie eine oder mehrere Futtermittelkomponenten, ausgewählt aus der Gruppe bestehend aus Proteinträgern, Kohlenhydratträgern, Raufutter, Silagen, Fetten, Vitaminen, Mineralstoffen und Spurenelementen, enthält.

Der Aufbau der Futterration für wiederkäuende landwirtschaftliche Nutztiere gestaltet sich grundsätzlich wie folgt:
Grundfuttermittel: das sind in der Regel wirtschaftseigene Futtermittel wie Weidegras und Graskonserven (Heu, Silage), Ackerfutter und Ackerfutterkonserven (Luzernensilage), Nebenprodukte (z.B. Biertreber).

Kraft- und Ergänzungsfuttermittel (leistungsabhängig): betriebseigenes Getreide, Nebenprodukte aus der Mehl-, Zucker- und Ölverarbeitung sowie zur Ergänzung vitaminisierte Mineralfutter. Heute üblich ist die Verwendung besonderer Ergänzungsfuttermittel für Milchvieh (z.B. Milchleistungsfutter) oder Ergänzungsfuttermittel für die Rindermast (allgemein als Kraftfutter oder Rindermastfutter bezeichnet).

Die tägliche Futteraufnahme für Grundfutter wird bei landwirtschaftlichen Nutztieren üblicherweise in Kilogramm Trockensubstanz (kg TS) angegeben. So beträgt etwa bei Milchkühen die Trockensubstanzaufnahmekapazität 3 bis 3,8 Prozent der Körpermasse, bei Mastbullen 1,6 bis 2,5 Prozent der Körpermasse. So liegt bei Milchkühen die tägliche Aufnahme an Trockensubstanz üblicherweise bei 15 bis 20 Kilogramm. Die täglichen Kraftfuttergaben liegen etwa für Milchviehkraftfutter (= Milchleistungsfutter) bei rund 5 bis 10 Kilogramm pro Tier und Tag, bei einem Mastleistungsfutter bei rund 2 bis 3 Kilogramm pro Tier und Tag.

Der oligomere Procyanidine enthaltende Tierfutterzusatz kann mit dem Tierfutter (beispielsweise Grundfutter, Milchleistungsfutter, Kraftfutter etc.) vermischt verabreicht werden, oder er kann on-top zu den jeweiligen Futterrationen zugegeben werden.

Das erfindungsgemäße Tierfutter enthält den wie oben beschrieben mit Eisenionen und/oder Eisenverbindungen komplexierte oligomere Procyanidine enthaltenden Tierfutterzusatz in Form von vermahlener Kiefernrinde in einer der Verabreichung von 0,1 bis 500 g vermahlener Kiefernrinde pro Tag pro Tier entsprechenden Menge. Alternativ enthält das erfindungsgemäße Tierfutter den wie oben beschrieben mit Eisenionen und/oder Eisenverbindungen komplexierte oligomere Procyanidine enthaltenden Tierfutterzusatz in Form von Kiefernrindenextrakt in einer der Verabreichung von 0,002 bis 100 g Kiefernrindenextrakt pro Tag pro Tier entsprechenden Menge.

Die in dem erfindungsgemäßen Tierfutter verwendete Menge mit eisenhaltiger Lösung behandelter, vermahlener Kiefernrinde kann im Bereich von 0,01 g bis 250 g vermahlene Rinde pro Kilogramm Futter-Trockensubstanz variieren. Beispielsweise enthält das Tierfutter vorzugsweise 0,01 g bis 100 g mit eisenhaltiger Lösung behandelte, vermahlene Kiefernrinde pro Kilogramm Milchleistungsfutter (Kraftfutter für Milchkühe), 0,03 g bis 250 g mit eisenhaltiger Lösung behandelte, vermahlene Kiefernrinde pro Kilogramm Mastleistungsfutter (Kraftfutter für Mastrinder) sowie 0,03 g bis 250 g mit eisenhaltiger Lösung behandelte, vermahlene Kiefernrinde pro Kilogramm Kraftfutter für kleine Wiederkäuer (z.B. Milchschafe, Fleischschafe, Wollschafe, Milchziegen, Fleischziegen).

Die in dem erfindungsgemäßen Tierfutter verwendete Menge mit eisenhaltiger Lösung behandelten Kiefernrindenextrakts kann im Bereich von 0,002 g bis 50 g Rindenextrakt pro Kilogramm Futter-Trockensubstanz variieren. Beispielsweise enthält das Tierfutter vorzugsweise 0,002 g bis 20 g mit Eisenlösung behandelten Kiefernrindenextrakt pro Kilogramm Milchleistungsfutter (Kraftfutter für Milchkühe), 0,002 g bis 50 g mit Eisenlösung behandelten Kiefernrindenextrakt pro Kilogramm Mastleistungsfutter (Kraftfutter für Mastrinder) sowie 0,002 g bis 50 g mit Eisenlösung behandelten Kiefernrindenextrakt pro Kilogramm Kraftfutter für kleine Wiederkäuer (z.B. Milchschafe, Fleischschafe, Wollschafe, Milchziegen, Fleischziegen).

Eine Vormischung zur Herstellung eines Tierfutters, wobei die Vormischung den mit Eisenionen und/oder Eisenverbindungen komplexierte oligomere Procyanidine enthaltenden Tierfutterzusatz zur Behandlung oder Prophylaxe von akuter oder subakuter Pansenazidose oder aus Pansenazidose resultierenden Zuständen zusammen mit
einer oder mehreren Futtermittelkomponenten, ausgewählt aus der Gruppe bestehend aus Proteinträgern, Kohlenhydratträgern, Raufutter, Silagen, Fetten, Vitaminen, Mineralstoffen und Spurenelementen, enthält, wird ebenfalls offenbart.

Die Erfindung betrifft auch die Verwendung eines erfindungsgemäßen Tierfutterzusatzes oder einer einen erfindungsgemäßen Tierfutterzusatz enthaltenden Vormischung zur Herstellung eines Tierfutters.

Gemäß einem anderen Aspekt betrifft die Erfindung die Verwendung von vermahlener Kiefernrinde, Kiefernrindenextrakt oder einer beliebigen Mischung davon, wie oben beschrieben mit eisenhaltiger Lösung behandelt, zur Herstellung eines Tierfutterzusatzes oder einer Tierfuttervormischung oder eines Tierfutters zur Behandlung oder Prophylaxe von akuter oder subakuter Pansenazidose oder aus Pansenazidose resultierenden Zuständen bei einem Wiederkäuer.

Wenn das Tierfutter die mit Eisenionen und/oder Eisenverbindungen komplexierten oligomeren Procyanidine in Form von vermahlener Kiefernrinde enthält, liegt die Dosierung bevorzugt im Bereich von 0,1 bis 500 g mit Eisenlösung behandelter vermahlener Kiefernrinde pro Tag pro Tier. Wenn das Tierfutter die mit Eisenionen und/oder Eisenverbindungen komplexierten oligomeren Procyanidine in Form von Kiefernrindenextrakt enthält, liegt die Dosierung bevorzugt im Bereich von 0,002 bis 100 g mit Eisenlösung behandeltem Kiefernrindenextrakt pro Tag pro Tier.

Gemäß einer weiteren Ausgestaltung der Erfindung kann der wie oben beschrieben mit Eisenionen und/oder Eisenverbindungen komplexierte oligomere Procyanidine enthaltende Kiefernrindenextrakt, vorzugsweise in Form eines wasserlöslichen Extrakts, auch dem Tränkwasser für Wiederkäuer beigemengt werden. Die in dem Tränkwasser verwendete Menge mit Eisenlösung behandelten Rindenextrakts kann im Bereich von 0,00008 g bis 100 g Extrakt pro Liter Wasser variieren. Alternativ dazu kann mit Eisenlösung behandelte vermahlene Kiefernrinde mit dem Tränkwasser aufgeschlämmt verabreicht werden. In diesem Fall kann die Dosierung beispielsweise zwischen 0,1 und 500 g pro Tier und Tag variieren.

Die Herstellung der Rindenextrakte kann nach an sich bekannten Verfahren erfolgen, wie Fest-flüssig-Extraktion mit Wasser, Heißwasser oder Dampf oder mit organischen Lösungsmitteln wie etwa Ethanol oder Methanol oder mit Lösungsmittelgemischen oder Gemischen solcher Lösungsmittel mit Wasser oder mittels Extraktion mit superkritischem CO₂ als Extraktionsmittel. Der Extraktionsgrad kann durch hydrolytische oder andere katalytisch abbauende (z.B. enzymatische, insbesondere zellulolytische Enzyme wie Zellulasen) Konditionen optimiert werden. Bevorzugt wird die Extraktion bei Temperaturen im Bereich von 50 °C bis 300 °C durchgeführt.

Zur Optimierung des Procyanidingehaltes wird Rinde vorzugsweise vermahlen und das so gewonnene Pulver in einem Perkolator mit 0,1 molarer Salzsäurelösung als Extraktionsmittel bei einer Temperatur von 100 °C extrahiert. Anschließend wird der Extrakt eingedampft und in Wasser aufgenommen.

Eine andere bevorzugte Extraktionsmethode ist die superkritische CO₂-Extraktion, bei der superkritisches CO₂ als Extraktionsmittel verwendet wird und bei einer Durchflussrate von 1200 bis 1400 Liter pro Stunde über einen Zeitraum von 4 Stunden bei einem Druck von 25 bis 30 MPa und einer Temperatur von 50 °C bis 70 °C extrahiert wird. Der Extrakt wird anschließend in Ethanol aufgenommen.

Eine Standardisierung des Gehaltes an oligomeren Procyanidinen kann mit Hilfe einer HPLC-Analyse aus den gewonnenen Extrakten erfolgen.

### Kurze Beschreibung der Zeichnungen

Im Folgenden wird die Erfindung anhand von Beispielen unter Bezugnahme auf die Zeichnungen näher erläutert. In den Zeichnungen zeigt die Figur 1 den Verlauf der Temperatur und des pH-Wertes im Pansen für (A) ein azidosesensibles Tier und für (B) ein azidoseunempfindliches Tier. Die Figur 2 zeigt den Einfluss der Verabreichung eines erfindungsgemäßen Tierfutterzusatzes auf die Azidosefrequenz im Fütterungsversuch mit Milchkühen. Die Figur 3 zeigt den Einfluss des erfindungsgemäßen Tierfutterzusatzes auf den Pansen-pH.

### BEISPIELE

### Beispiel 1: In-vitro-Tests mit oligomeren Procyanidinen

Gesunden, unbehandelten, pansenfistulierten Ochsen wurde Pansensaft entnommen, abzentrifugiert und der Überstand sofort tiefgefroren. 10 ml des aufgetauten Pansensaftes wurden mit 0,5 g der erfindungsgemäßen Substanz vermengt und für 60 min, 90 min, 120 min, 150 min, 180 min, 210 min und 240 min bei 39°C mittels Magnetrührer gerührt. Der Überstand wurde zum jeweils angegebenen Zeitpunkt abpipettiert und die Amylaseaktivität gemessen. Dazu wurde ein handelsübliches photometrisches-System verwendet, welches üblicherweise zur Bestimmung der Amylaseaktivität im Blutserum verwendet wird.

**Tabelle 1: Amylasemessung in Pansensaft**

| Zeitpunkt | Pansensaft ohne Versuchssubstanz (Kontrolle) | Pansensaft mit Zusatz oligomerer Procyanidine |
|---|---|---|
| [min] | [U/l] | [U/l] |
| 60 | 209 | 129 |
| 90 | 212 | 153 |
| 120 | 197 | 149 |
| 150 | 211 | 159 |
| 180 | 204 | 153 |
| 210 | 194 | 163 |
| 240 | 181 | 167 |

Wie anhand der Tabelle 1 deutlich wird, verursachen die verwendeten oligomeren Procyanidine eine Hemmung der Amylaseaktivität.

### Beispiel 2: In-vitro-Tests mit einem oligomeren Procyanidin-Eisen-Komplex

Einem gesunden, unbehandelten, pansenfistulierten Ochsen wurde Pansensaft entnommen, abzentrifugiert und der Überstand sofort tiefgefroren. 10 ml des aufgetauten Pansensaftes wurden mit 0,5 g der erfindungsgemäßen Substanz, vorliegend als eisenhaltiger Komplex, vermengt und für 60 min, 90 min, 120 min und 240 min bei 39°C mittels Magnetrührer gerührt. Der Überstand wurde zum jeweils angegebenen Zeitpunkt abpipettiert und der photometrischen Amylaseaktivitätsmessung zugeführt.

**Tabelle 2: Amylasemessung in Pansensaft unter Verwendung eines oligomeren Procyanidin-Eisen-Komplexes**

| Zeitpunkt | Pansensaft ohne Versuchssubstanz (Kontrolle) | Pansensaft mit Zusatz oligomerer Procyanidin-Eisen-Komplex gemäß der Erfindung |
|---|---|---|
| [min] | [U/l] | [U/l] |
| 0 | 333 | 333 |
| 60 | 265 | 2 |
| 90 | 256 | 1 |
| 120 | 249 | 0 |
| 240 | 241 | 0 |

Aus der Tabelle 2 ist ersichtlich, dass die erfindungsgemäße Substanz, vorliegend als eisenhaltiger Komplex, eine Hemmung der Amylaseaktivität verursacht, wobei die amylasehemmende Wirkung deutlicher ausgeprägt ist, als bei dem *in-vitro*-Test im Beispiel 1.

### Beispiel 3: In-vivo-Versuche mit Mastbullen

Wie bereits oben ausgeführt wurde, ist es aus physiologischer Sicht wichtig, dass bei Tieren, die keine Pansenazidose entwickeln oder für diese nicht empfänglich sind, keine Hemmung der Pansenamylase stattfindet. Eine solche Hemmung kann bei gesunden Tieren zu einer Entgleisung der Pansenfermentation führen, was seinerseits gesundheitliche Störungen und Leistungsdepression bewirkt.

Dieses Beispiel zeigt, dass die erfindungsgemäße Versuchssubstanz (z.B. Kiefernrinde bzw. Kiefernrindenextrakt), abhängig vom Extraktionsgrad, einen Wirkungsmechanismus zeigt, der durch einen Abfall des pH Wertes (d.h. einen Säureanstieg) bedingt ist. Umfangreiche Untersuchungen haben ergeben, dass die oligomeren Procyanidine der Kiefernrinde durch einen Säureanstieg (i.e. pH-Abfall) in einen Bereich, in dem mit der Entstehung der (subakuten) Pansenazidose zu rechnen ist, freigesetzt und damit aktiv werden. Die freigesetzte Menge und die Freisetzungsgeschwindigkeit können durch den Extraktionsgrad der Rinde beeinflusst werden.

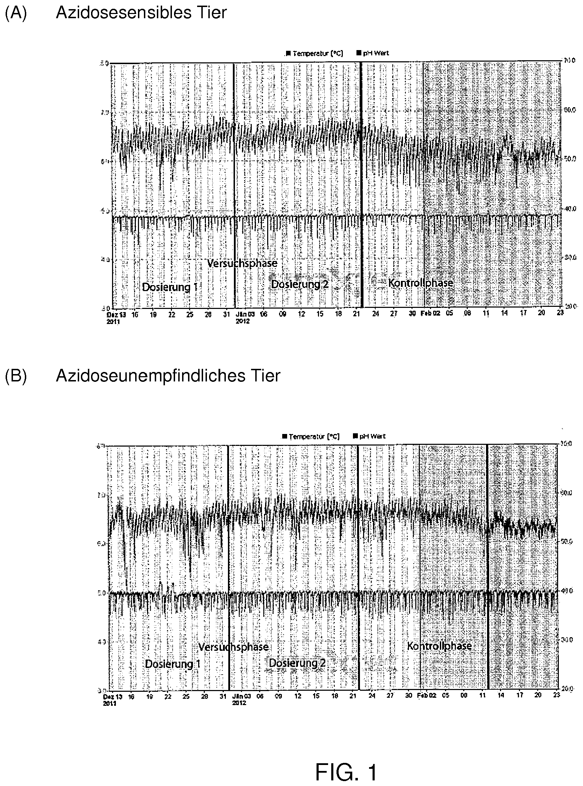

Vermahlene Rinde hat im Vergleich zu Rindenextrakten die größte Verzögerung der Wirkstofffreisetzung. Sie ist daher in der Praxis besonders vorteilhaft, wenn es darum geht, gezielt bei azidosesensiblen Tieren über die Hemmung der Pansenamylase einen pH-Abfall zu vermeiden, ohne dass man dabei das Risiko eingeht, bei gesunden oder nicht sensiblen Tieren die Pansenphysiologie unnötig zu stören (siehe Figur 1A und 1B).

Diese Erkenntnisse wurden in umfangreichen Versuchen unter Verwendung von Pansenboli gewonnen, wobei der Einfluss der oligomeren Procyanidine bei Mastbullen untersucht wurde. Bei den verwendeten Pansenboli handelt es sich um Messsensoren, wobei jeweils ein Sensor in den Pansen des Versuchstieres gelegt wird. Der Sensor misst im Abstand weniger Minuten kontinuierlich sowohl den pH-Wert als auch die Temperatur und speichert diese Daten. Eine externe Datenspeicherstation ruft diese Daten mehrmals täglich ab und leitet sie an einen Computer zur Datenauswertung weiter.

Mastbullen wurden deshalb für die Versuche herangezogen (und Milchkühen vorgezogen), weil im Fall von Milchkühen die Verwertung von flüchtigen Fettsäuren für die Milchbildung je nach Laktationsstadium und Milchmenge unterschiedlich ist und somit einen weiteren Einflussfaktor darstellen würde.

Die Versuche mit Pansenboli wurden an sechs Mastochsen über einen Zeitraum von insgesamt neun Wochen im lateinischen Quadrat durchgeführt. Zum Einsatz kam einerseits Versuchsfutter, welches oligomere Procyanidine in zwei verschiedenen Dosierungen enthielt, sowie Kontrollfutter ohne Beimischung eines erfindungsgemäßen Tierfutterzusatzes. Das Versuchschema ist in der nachstehenden Tabelle 3 dargestellt.

**Tabelle 3:**

| | Kontrollfutter | Versuchsfutter | Versuchsfutter |
|---|---|---|---|
| | | Dosierung 1 | Dosierung 2 |
| Periode 1 (3 Wochen) | Tier 1 | Tier 3 | Tier 5 |
| | Tier 2 | Tier 4 | Tier 6 |
| Periode 2 (3 Wochen) | Tier 5 | Tier 1 | Tier 3 |
| | Tier 6 | Tier 2 | Tier 4 |
| Periode 3 (3 Wochen) | Tier 3 | Tier 5 | Tier 1 |
| | Tier 4 | Tier 6 | Tier 2 |

Den Tieren wurde ein Tierfutter aus Kraftfutter, Maissilage und Heu verabreicht, wobei das Kraftfutter zu gleichen Teilen aus Gerste, Weizen und Soja HP (d.h. Hoch-Protein-Sojaextraktionsschrot) bestand. Die Zusammensetzung des Futters ist in der nachstehenden Tabelle 4 angeführt. In den Versuchsperioden wurde dem Tierfutter der erfindungsgemäße Tierfutterzusatz on-top zugemischt. Der oligomere Procyanidine enthaltende Futterzusatz wurde im Falle des Versuchsfutters in zwei verschiedenen Dosierungen (15 g pro Tier pro Tag bzw. 30 g pro Tier pro Tag) gefüttert.

**Tabelle 4:**

| | | |
|---|---|---|
| Maissilage | kg | 9,09 |
| Kraftfutter | kg | 2,73 |
| Heu | kg | 0,69 |
| Kraftfutterzusammensetzung: 33,3 % Gerste, 33,3 % Weizen, 33,3 % Soja HP | | |

Die Fig. 1 zeigt den mit den Pansenboli gemessenen Verlauf des pH-Wertes und der Temperatur im Pansen, und zwar für ein azidosesensibles Tier (Fig. 1A) und für ein azidoseunempfindliches Tier (Fig. 1B).

Bei dem azidosesensiblen Tier kam es durch die Provokationsfütterung mit dem stärkereichen Tierfutter in der Kontrollphase, d.h. ohne Verabreichung des erfindungsgemäßen Futterzusatzes, zu einem Absinken des pH-Wertes. Dagegen zeigten sich während der Versuchsphase, d.h. für die Dauer der Verabreichung des erfindungsgemäßen Tierfutterzusatzes, höhere pH-Werte. Dies bestätigt die Wirksamkeit des erfindungsgemäßen Tierfutterzusatzes zur Behandlung oder Prophylaxe von Pansenazidose.

Im Falle des azidoseunempfindlichen Tieres zeigte sich in der Kontrollphase, d.h. ohne den erfindungsgemäßen Tierfutterzusatz, kein Absinken des Pansen-pH. Es war keine Störung der Pansenphysiologie in der Versuchsphase feststellbar. Dieses Ergebnis bestätigt den weiter oben beschriebenen Wirkungsmechanismus des erfindungsgemäßen Tierfutterzusatzes. Bei gesunden oder azidoseunempfindlichen Tieren wird die Pansenphysiologie durch den erfindungsgemäßen Tierfutterzusatz nicht gestört, da das zum Auslösen seiner Wirkung nötige Abfallen des pH-Wertes im Pansen nicht erfolgt.

### Beispiel 4: In-vivo-Versuche mit Milchkühen

Bei diesem Versuch handelte es sich um einen Feldversuch in einem Milchviehbetrieb. Während der Versuchsphase wurde allen laktierenden Milchkühen der Herde 30 g Ulmenrinde zur stallüblichen Ration on-top zugefüttert. Die Daten der stallüblichen Routineuntersuchungen zur Herdengesundheit der Versuchsphase wurden mit früheren Daten verglichen, die als Kontrolldaten (ohne Zufütterung von Rinde) bewertet wurden. Als Versuchstiere konnten 40 laktierende Milchkühe ausgewertet werden. Das verabreichte Tierfutter war das im Beispiel 4 genannte Futter. Die Dosierung des erfindungsgemäßen Tierfutterzusatzes war 30 g Ulmenrinde pro Tier und Tag.

Im Rahmen dieser Versuche bei Milchkühen konnte festgestellt werden, dass der erfindungsgemäße Tierfutterzusatz insbesondere in kritischen Phasen der Laktation zu einer höheren Milchleistung führte. Wie die nachstehende Tabelle 4 deutlich zeigt, war dieser Effekt überraschenderweise nicht mit der aus fachlicher Sicht zu erwartenden Depression des Milchfettgehaltes verbunden. Die Wirkung des erfindungsgemäßen Tierfutterzusatzes steht somit in krassem Gegensatz zur Lehre des Standes der Technik, die besagt, dass ein Anstieg der Milchleistung stets mit einem Abfall des Milchfettgehaltes einhergeht.

**Tabelle 5: Milchmenge und Milchfettgehalte azidosegefährdeter Tiere**

| | Routinemäßige Herdenuntersuchung | | |
|---|---|---|---|
| | Zeitpunkt 1 Kontrolle | Zeitpunkt 2 Kontrolle | Zeitpunkt 3 Versuch |
| Anzahl der Kühe über 35 kg Milchleistung | 16 | 16 | 11 |
| Milchmenge in kg | 39,9 | 38,9 | 41,3 |
| Milchfettgehalt in % | 4,21 | 4,23 | 4,26 |
| | | | |
| Anzahl der erstlaktierenden Kühe im Laktationszeitraum Tag 1 bis 100 | 3 | 4 | 4 |
| Milchmenge in kg | 23,2 | 29,5 | 33,6 |
| Milchfettgehalt in % | 4,71 | 4,23 | 4,33 |
| | | | |
| Anzahl der Kühe ab 2. Laktation im Laktationszeitraum Tag 1 bis 100 | 9 | 10 | 6 |
| Milchmenge in kg | 41,0 | 39,8 | 43,3 |
| Milchfettgehalt in % | 4,06 | 4,11 | 4,26 |

Interessant sind auch die routinemäßig erhobenen Parameter zur Herdengesundheit: Der Harnstoffgehalt der Milch dient als Indikator der Eiweißversorgung. Der Eiweißgehalt der Milch ist ein Indikator für die Energieversorgung des Tieres. Die beiden Parameter sind gemeinsam als Harnstoff/Eiweiß zu beurteilen und werden in neun Klassen eingeteilt. Der Wert soll im mittleren Feld (Klasse 5) liegen. Abweichungen bedeuten eine Über- oder Unterversorgung mit Eiweiß und/oder mit Energie.

Stoffwechselkontrolle Harnstoff/Eiweiß (Harnstoffklasse, HKI) ohne erfindungsgemäßen Tierfutterzusatz:
Maximal 47,5% der Tiere waren in der optimalen Klasse 5 kategorisiert, bis zu 42,5% der Tiere waren in den ungünstigen Klassen 2 (Energiemangel) bzw. 7, 8 und 9 (Proteinüberschuss und/oder Energieüberschuss).

Stoffwechselkontrolle Harnstoff/Eiweiß (HKl) mit erfindungsgemäßem Tierfutterzusatz: 70,7% der Tiere waren in der optimalen Klasse 5 kategorisiert, 9,8% in Klasse 2, 17,1% in Klasse 8, kein Tier wurde als Klasse 9 ausgewiesen.

Der Parameter Stoffwechselkontrolle Energieversorgung beschreibt die Energieversorgung des Tieres über den Eiweißgehalt der Milch. Ein Eiweißgehalt von 3,2 % bis 3,8 % wird als normal eingestuft, darüber liegt eine Energieüberversorgung, darunter eine Energieunterversorgung vor. Prinzipiell ist ein Energiemangel von einem Abfallen des Milcheiweißgehaltes begleitet.

Stoffwechselkontrolle Energieversorgung ohne erfindungsgemäßen Tierfutterzusatz: Rund 58% der Tiere lagen im Bereich "normal", bis zu 35% der Tiere im Bereich "Energieüberschreitung".

Stoffwechselkontrolle Energieversorgung mit erfindungsgemäßem Tierfutterzusatz: Rund 73% der Tiere lagen im Bereich "normal", lediglich 17% der Tiere im Bereich "Energieüberschreitung".

Die deutliche Verbesserung dieser Parameter, die die Pansengesundheit und Stoffwechsellage widerspiegeln, deutet darauf hin, dass der erfindungsgemäße Tierfutter- bzw. Tränkwasserzusatz tatsächlich zu einer Verbesserung der Pansenphysiologie und damit der gesamten Stoffwechselsituation der Tiere führt.

### Beispiel 5: Erfindungsgemäßes Tierfutter für Milchkühe

Dieses Beispiel gibt eine Zusammensetzung eines Ausgleichskraftfutters für Milchkühe an.

**Tabelle 6:**

| Rohstoff | Trockenmasse | Anteil in % |
|---|---|---|
| Mais | 880 | 10,00 |
| Gerste | 870 | 15,00 |
| Weizen | 870 | 11,50 |
| Sojaschrot | 870 | 10,00 |
| Maiskraftfutter | 880 | 5,00 |
| Rapsschrot | 886 | 15,00 |
| Weizenfuttermehl | 882 | 5,00 |
| Weizenkleie | 880 | 10,00 |
| Trockenschnitte | 906 | 15,00 |
| Futterkalk | 980 | 1,20 |
| Kiefern- oder Ulmenrinde | 920 | 0,30 |
| Mineralstoffmischung | 900 | 2,00 |
| | | 100,00 |

**Tabelle 7:**

| Nährstoff | Einheit | Gehalt |
|---|---|---|
| Trockensubstanz | % | 88,34 |
| Nettoenergie-Laktation (NEL) | MJ | 6,59 |
| ME-Wiederkäuer*) | MJ | 10,50 |
| Rohprotein | % | 17,72 |
| Rohfaser | % | 8,14 |
| Calcium | % | 0,89 |
| Phosphor | % | 0,60 |
| Natrium | % | 0,36 |
| Magnesium | % | 0,35 |
| Ca:P | | =1,49:1 |
| Milch aus Protein | I | 2,09 |
| Milch aus NEL | I | 2,10 |

| | | |
|---|---|---|
| *) Metabolische Energie: Energiebewertungsschema für Wiederkäuer | | |

### Beispiel 6: Erfindungsgemäßes Tierfutter für Rindermast

Dieses Beispiel gibt eine Zusammensetzung eines Kraftfutters für Mastrinder an.

**Tabelle 8:**

| Rohstoff | Trockenmasse | Anteil in % |
|---|---|---|
| Gerste | 870 | 9,80 |
| Weizen | 870 | 10,00 |
| Sojaschrot | 870 | 19,00 |
| Maiskraftfutter | 880 | 5,00 |
| Rapsschrot | 886 | 20,00 |
| Weizenfuttermehl | 882 | 5,00 |
| Weizenkleie | 880 | 10,00 |
| Trockenschnitte | 906 | 15,00 |
| Futterkalk | 980 | 2,00 |
| Viehsalz | 900 | 0,20 |
| Kiefern- oder Ulmenrinde | 920 | 1,00 |
| Mineralstoffmischung | 900 | 3,00 |
| | | 100,00 |

**Tabelle 9:**

| Nährstoff | Einheit | Gehalt |
|---|---|---|
| Trockensubstanz | % | 88,39 |
| ME-Wiederkäuer*) | MJ | 10,29 |
| Rohprotein | % | 22,01 |
| Rohfaser | % | 8,90 |
| Calcium | % | 1,74 |
| Phosphor | % | 0,76 |
| Calcium : Phosphor | | = 2,29:1 |
| Natrium | % | 0,31 |
| Magnesium | % | 0,44 |
| Vitamin D | IE | 3900 |
| Vitamin E | mg | 19 |
| Vitamin B1 | mg | 1,56 |
| ME-W*) /kg Trockenmasse | MJ | 11,65 |
| % Rohfaser/kg Trockenmasse | | 10,07 |

| | | |
|---|---|---|
| *) Metabolische Energie: Energiebewertungsschema für Wiederkäuer | | |

Ein solches Futter ist dem Grunde nach auch für kleine Wiederkäuer wie Schafe in der Milch-, Fleisch- und Wollproduktion und Ziegen in der Milch- und Fleischproduktion geeignet.

### Beispiel 7: In-vivo-Versuch mit Milchkühen

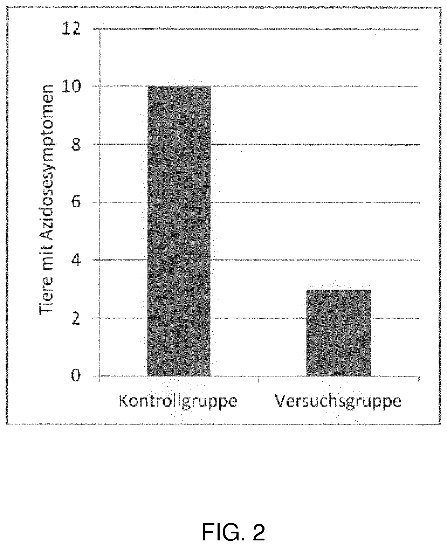

Im Rahmen eines weiteren Fütterungsversuches mit laktierenden Milchkühen wurde die erfindungsgemäße Substanz, in der Ausführungsform eines eisenhaltigen Komplexes mit dem Verhältnis Eisen zu oligomeren Procyanidinen von 1:500, in einer Dosierung von 25 g pro Tier pro Tag verabreicht. Insgesamt nahmen 54 laktierende Milchkühe (Rasse: Deutsche Holstein) an diesem Versuch teil. Die 27 Tiere der Versuchsgruppe erhielten die erfindungsgemäße Substanz in der angegebenen Dosierung über einen Zeitraum von 14 Tagen über das Milchleistungsfutter. Die Kontrollgruppe bestand ebenfalls aus 27 Tieren und wurde mit dem gleichen Milchleistungsfutter ohne Zusatz der erfindungsgemäßen Substanz gefüttert. Anhand klinischer Parameter und anhand von Stoffwechselparametern wurde die Azidosefrequenz der Tiere gemessen. Wie aus der Figur 2 ersichtlich ist, konnte in der Versuchsgruppe eine Verringerung der Azidosefrequenz um 70% gemessen werden. Dies stellt eine weitere Optimierung des Effektes der erfindungsgemäßen Substanz als Kombination von Eisenionen bzw. Eisenverbindungen mit oligomeren Procyanidinen dar, zumal dieser Effekt mit einer um ca. 20% reduzierten Einsatzdosierung erreicht werden konnte.

### Beispiel 8: Einfluss des erfindungsgemäßen Tierfutterzusatzes auf den Pansen-pH

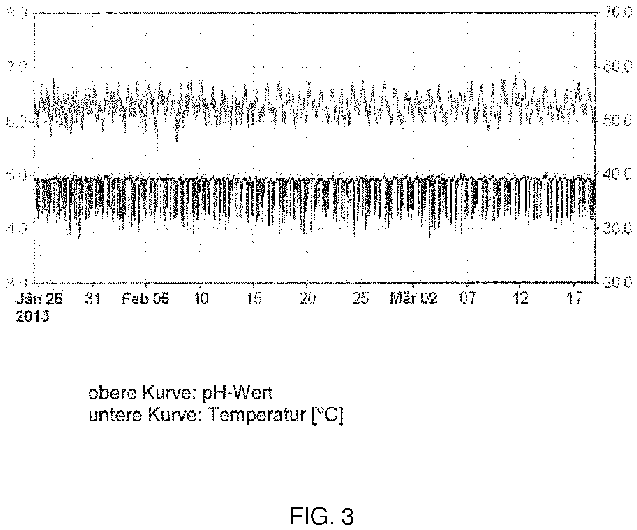

Ein weiterer besonderer Vorteil der erfindungsgemäßen Substanz, vorliegend als Komplex aus Eisenionen bzw. Eisenverbindungen mit oligomeren Procyanidinen, ist die Tatsache, dass kleine Schwankungen des Pansen-pH über den Tagesverlauf ausgeglichen werden können. Dies geht aus dem in der Fig. 3 gezeigten Messprotokoll hervor, in dem der Effekt der erfindungsgemäßen Substanz auf den Pansen-pH überprüft wurde. Nach Verabreichung der erfindungsgemäßen Testsubstanz von Beispiel 7 (ab 15. Februar) in einer Dosierung von 25 g pro Tier pro Tag wurde im Messprotokoll eine deutlich geringere Varianz der einzelnen Tagesschwankungen des pH-Wertes verzeichnet. Größere Varianzen dieser Tagesschwankungen sind vermutlich aus fachlicher Sicht ein wesentlicher Schadfaktor für die Gesundheit der Pansenschleimhaut und in weiterer Folge für die Sensibilität eines Tieres für ein subklinisches Azidosegeschehen.

### Beispiel 9: Einfluss des erfindungsgemäßen Tierfutterzusatzes auf die Ausscheidung von Aflatoxinen in die Milch

Im Rahmen eines Fütterungsversuches in einem Milchviehbetrieb wurde der Einfluss der erfindungsgemäßen Substanz auf die Ausscheidung von Aflatoxinen in die Milch überprüft. Im Rahmen des Versuches wurden die Tiere des Betriebes in eine Kontrollgruppe und eine Versuchsgruppe mit jeweils 50 laktierenden Milchkühen unterteilt. Der Versuchsgruppe wurde über einen Zeitraum von 14 Tagen über das Milchleistungsfutter die erfindungsgemäße Substanz in einer Dosierung von 30 g pro Tier pro Tag zugefüttert. Am Ende der Versuchsperiode wurde der Aflatoxingehalt der Milchproben in beiden Gruppen gemessen, in den Milchsammelproben der Kontrollgruppe war der Aflatoxingehalt bei 39,2 ppt ("parts per trillion") gelegen, in den Milchsammelproben der Versuchsgruppe bei 11 ppt. Dies stellt eine Reduktion um 72 Prozent dar.

**Tabelle 10: Aflatoxin-Versuch 1**

| Aflatoxingehalt in ppt | | |
|---|---|---|
| Kontrollgruppe | Versuchsgruppe | Reduktion in % |
| 39,2 | 11,0 | 72 |

Weiters wurden in diesem Fütterungsversuch auch die Parameter des Azidoserisikos beurteilt, und es konnte festgestellt werden, dass es zu einer signifikanten Besserung der Azidosefrequenz und der daraus resultierenden Stoffwechselparameter gekommen ist. So konnte der für das Azidosegeschehen relevante Parameter, nämlich der Fett-Eiweiß-Quotient, in der Versuchsgruppe erhöht werden. Dies bestätigt den unmittelbaren Zusammenhang zwischen der Senkung der Azidosefrequenz und dem daraus resultierenden verbesserten Abbau von Aflatoxinen im Pansen bzw. der daraus resultierenden verminderten Ausscheidung von Aflatoxinen in die Milch.

Im Rahmen eines weiteren Fütterungsversuches mit laktierenden Milchkühen wurde die erfindungsgemäße Substanz, vorliegend in Form eines eisenhaltigen Komplexes (Verhältnis Fe zu oligomere Procyanidine 1:350), in einer Dosierung von 25 g pro Tier pro Tag eingesetzt. Dieser Versuch wurde mit insgesamt 72 laktierenden Milchkühen über einen Zeitraum von 21 Tagen durchgeführt. Die 36 Tiere der Versuchsgruppe erhielten die erfindungsgemäße Substanz in der angegebenen Dosierung während der gesamten Versuchsperiode über das Milchleistungsfutter. Am Ende der Versuchsperiode wurde der Aflatoxingehalt in den Milchsammelproben der Gruppen gemessen. In der Kontrollgruppe lag der gemessene Wert bei 75,2 ppt, in der Versuchsgruppe bei 19,6 ppt. Dies stellt eine Reduktion um 74 Prozent dar.

**Tabelle 11: Aflatoxin-Versuch 2**

| Aflatoxingehalt in ppt | | |
|---|---|---|
| Kontrollgruppe | Versuchsgruppe | Reduktion in % |
| 75,2 | 19,6 | 74 |

## Patentansprüche

1. Tierfutterzusatz zur Verwendung bei der Behandlung oder Prophylaxe von akuter oder subakuter Pansenazidose oder aus Pansenazidose resultierenden Zuständen bei einem Wiederkäuer, als Tierfutterzusatz, **dadurch gekennzeichnet, dass** es mit Eisenionen und/oder Eisenverbindungen komplexiert vorliegende oligomere Procyanidine enthält, wobei das Verhältnis zwischen Eisenionen und/oder Eisenverbindungen und oligomeren Procyanidinen zwischen 1:10 und 1:20 000, vorzugsweise zwischen 1:100 und 1:8 000 liegt und wobei die oligomeren Procyanidine in Form von vermahlener Kiefernrinde, Kiefernrindenextrakt oder einer beliebigen Mischung davon enthalten sind.

2. Tierfutter für Wiederkäuer zur Verwendung bei der Behandlung oder Prophylaxe von Pansenazidose oder aus Pansenazidose resultierenden Zuständen, enthaltend zwischen 0,01 g und 250 g Kiefernrinde pro Kilogramm Tierfutter-Trockengewicht oder zwischen 0,002 g und 50 g Kiefernrindenextrakt pro Kilogramm Tierfutter-Trockengewicht zusammen mit einer oder mehreren Futtermittelkomponenten, ausgewählt aus der Gruppe bestehend aus Proteinträgern, Kohlenhydratträgern, Raufutter, Silagen, Fetten, Vitaminen, Mineralstoffen und Spurenelementen, wobei das Tierfutter mit Eisenionen und/oder Eisenverbindungen komplexiert vorliegende oligomere Procyanidine enthält, wobei die oligomeren Procyanidine in Form von vermahlener Kiefernrinde, Kiefernrindenextrakt oder einer beliebigen Mischung davon enthalten sind, und wobei das Verhältnis zwischen Eisenionen und/oder Eisenverbindungen und oligomeren Procyanidinen zwischen 1:10 und 1:20 000, vorzugsweise zwischen 1:100 und 1:8 000 liegt.

3. Tierfutter zur Verwendung nach Anspruch 2, **dadurch gekennzeichnet, dass** es 0,01 g bis 250 g vermahlene Kiefernrinde, die mit eisenhaltiger Lösung behandelt wurde, pro Kilogramm Futter-Trockensubstanz enthält.

4. Tierfutter zur Verwendung nach Anspruch 2, **dadurch gekennzeichnet, dass** es 0,002 g bis 50 g Kiefernrindenextrakt, der mit eisenhaltiger Lösung behandelt wurde, pro Kilogramm Futter-Trockensubstanz enthält.

5. Vermahlene Kiefernrinde, Kiefernrindenextrakt oder eine beliebige Mischung davon, die/der mit eisenhaltiger Lösung behandelt wurde, wobei das Verhältnis zwischen Eisenionen und/oder Eisenverbindungen und den in der Kiefernrinde und/oder dem Kiefernrindenextrakt enthaltenen oligomeren Procyanidinen zwischen 1:10 und 1:20000, vorzugsweise zwischen 1:100 und 1:8000, liegt, wobei die oligomeren Procyanidine als eisenhaltiger Komplex vorliegen, zur Verwendung bei der Behandlung oder Prophylaxe von akuter oder subakuter Pansenazidose oder aus Pansenazidose resultierenden Zuständen bei einem Wiederkäuer.

6. Vermahlene Kiefernrinde, Kiefernrindenextrakt oder eine beliebige Mischung davon zur Verwendung nach Anspruch 5, wobei der Wiederkäuer 0,1 bis 500 g vermahlene Kiefernrinde, die mit eisenhaltiger Lösung behandelt wurde, pro Tag erhält.

7. Vermahlene Kiefernrinde, Kiefernrindenextrakt oder eine beliebige Mischung davon zur Verwendung nach Anspruch 5, wobei der Wiederkäuer 0,002 bis 100 g Kiefernrindenextrakt, der mit eisenhaltiger Lösung behandelt wurde, pro Tag erhält.

8. Vermahlene Kiefernrinde, Kiefernrindenextrakt oder eine beliebige Mischung davon zur Verwendung nach Anspruch 5, wobei die mit eisenhaltiger Lösung behandelte vermahlene Kiefernrinde, der mit eisenhaltiger Lösung behandelte Kiefernrindenextrakt, oder eine beliebige Mischung davon, als Tränkwasserzusatz vorliegt.

9. Vermahlene Kiefernrinde, Kiefernrindenextrakt oder eine beliebige Mischung davon zur Verwendung nach Anspruch 8, wobei der Tränkwasserzusatz 0,00008 g bis 100 g Kiefernrindenextrakt, der mit eisenhaltiger Lösung behandelt wurde, pro Liter enthält.

10. Vermahlene Kiefernrinde, Kiefernrindenextrakt oder eine beliebige Mischung davon zur Verwendung nach einem der Ansprüche 5 bis 9, wobei die aus Pansenazidose resultierenden Zustände bei einem Wiederkäuer ausgewählt sind aus der Gruppe bestehend aus pH-Absenkung im Pansen, Entgleisung der Pansenflora, Sistieren der Wiederkautätigkeit, Abfall der Milchmenge, Abfall des Milchfettgehaltes, chronischer Stoffwechselbelastung, Belastung des Leberstoffwechsels, Unfruchtbarkeit, verlängerter Remontierungsrate, geringerer Kälberzahl und entzündlichen Klauenerkrankungen.

11. Verwendung von vermahlener Kiefernrinde, Kiefernrindenextrakt oder einer beliebigen Mischung davon, insbesondere von oligomeren Procyanidinen aus Kiefernrinde, Kiefernrindenextrakt oder einer beliebigen Mischung davon, die/der mit eisenhaltiger Lösung behandelt wurde zur Reduktion der Aflatoxinausscheidung in der Milch eines Wiederkäuers, wobei das Verhältnis zwischen komplexierbaren Eisenionen und/oder komplexierbaren Eisenverbindungen und den in der Kiefernrinde und/oder dem Kiefernrindenextrakt enthaltenen oligomeren Procyanidinen zwischen 1:10 und 1:20000, vorzugsweise zwischen 1:100 und 1:8000, liegt, und wobei die oligomeren Procyanidine als eisenhaltiger Komplex vorliegen.

## Claims

1. An animal feed additive for use in the treatment or prophylaxis of acute or subacute ruminal acidosis or conditions resulting from ruminal acidosis in a ruminant as an animal feed additive, **characterized in that** it contains oligomeric procyanidins complexed with iron ions and/or iron compounds, wherein the ratio between the iron ions and/or iron compounds and the oligomeric procyanidins is in the range 1:10 to 1:20000, preferably in the range 1:100 to 1:8000, and wherein the oligomeric procyanidins are in the form of ground pine bark, pine bark extract or any mixture thereof.

2. An animal feed for ruminants for use in the treatment or prophylaxis of ruminal acidosis or conditions resulting from ruminal acidosis, containing in the range 0.01 g to 250 g of pine bark per kilogram of feed dry matter or in the range 0.002 g to 50 g of pine bark extract per kilogram of feed dry matter together with one or more feed material components selected from the group consisting of protein carriers, carbohydrate carriers, forage, silage, fats, vitamins, minerals and trace elements, wherein the animal feed contains oligomeric procyanidins complexed with iron ions and/or iron compounds, wherein the oligomeric procyanidins are in the form of ground pine bark, pine bark extract or any mixture thereof, and wherein the ratio between the iron ions and/or iron compounds and the oligomeric procyanidins is in the range 1:10 to 1:20000, preferably in the range 1:100 to 1:8000.

3. The animal feed for use as claimed in claim 2, **characterized in that** it contains 0.01 g to 250 g of ground pine bark per kilogram of feed dry matter which has been treated with an iron-containing solution.

4. The animal feed for use as claimed in claim 2, **characterized in that** it contains 0.002 g to 50 g of pine bark extract per kilogram of feed dry matter which has been treated with an iron-containing solution.

5. Ground pine bark, pine bark extract or any mixture thereof which has been treated with an iron-containing solution, wherein the ratio between the iron ions and/or iron compounds and the oligomeric procyanidins contained in the pine bark and/or in the pine bark extract is in the range 1:10 to 1:20000, preferably in the range 1:100 to 1:8000, wherein the oligomeric procyanidins are in the form of an iron-containing complex, for use in the treatment or prophylaxis of acute or subacute ruminal acidosis or conditions resulting from ruminal acidosis in a ruminant.

6. Ground pine bark, pine bark extract or any mixture thereof, for use as claimed in claim 5, wherein the ruminant receives 0.1 to 500 g per day of ground pine bark which has been treated with an iron-containing solution.

7. Ground pine bark, pine bark extract or any mixture thereof, for use as claimed in claim 5, wherein the ruminant receives 0.002 to 100 g per day of pine bark extract which has been treated with an iron-containing solution.

8. Ground pine bark, pine bark extract or any mixture thereof, for use as claimed in claim 5, wherein the ground pine bark treated with an iron-containing solution, the pine bark extract treated with an iron-containing solution, or any mixture thereof is in the form of a drinking water additive.

9. Ground pine bark, pine bark extract or any mixture thereof, for use as claimed in claim 8, wherein the drinking water additive contains 0.00008 g to 100 g per litre of pine bark extract which has been treated with an iron-containing solution.

10. Ground pine bark, pine bark extract or any mixture thereof, for use as claimed in one of claims 5 to 9, wherein the conditions resulting from ruminal acidosis in a ruminant are selected from the group consisting of a reduction of the pH in the rumen, disruption of the ruminal flora, suspension of rumination, a drop in milk production, a drop in milk fat content, chronic metabolic problems, liver metabolism problems, infertility, a longer re-stocking rate, a smaller number of calves and inflammatory hoof disease.

11. Use of ground pine bark, pine bark extract or any mixture thereof, in particular of oligomeric procyanidins from pine bark, pine bark extract or any mixture thereof which has been treated with an iron-containing solution, for the reduction of aflatoxin excretion in the milk of a ruminant, wherein the ratio between the complexable iron ions and/or complexable iron compounds and the oligomeric procyanidins contained in the pine bark and/or in the pine bark extract is in the range 1:10 to 1:20000, preferably in the range 1:100 to 1:8000, wherein the oligomeric procyanidins are in the form of an iron-containing complex.

## Revendications

1. Additif pour alimentation animale, destiné à être utilisé pour le traitement ou la prophylaxie de l'acidose ruménale aiguë ou subaiguë ou d'états résultant de l'acidose ruménale chez un ruminant, sous la forme d'additif pour alimentation animale, **caractérisé en ce qu'**il contient des procyanidines oligomères présentes sous la forme complexée avec des ions ferriques et/ou des composés ferreux, le rapport des ions ferriques et/ou composés ferreux aux procyanidines oligomères se situant entre 1 : 10 et 1 : 20000, de préférence entre 1 : 100 et 1 : 8000 et les procyanidines oligomères étant contenues sous la forme d'écorce de pin moulue, d'extrait d'écorce de pin ou d'un mélange quelconque de ces derniers.

2. Alimentation animale pour ruminants, destinée à être utilisée pour le traitement ou la prophylaxie de l'acidose ruménale ou d'états résultant de l'acidose ruménale, contenant entre 0,01 g et 250 g d'écorce de pin par kilogramme de masse sèche d'alimentation animale ou entre 0,002 g et 50 g d'extrait d'écorce de pin par kilogramme de masse sèche d'alimentation animale, en commun avec un ou plusieurs composants d'alimentation animale, choisi(s) dans le groupe composé des protéagineux, des porteurs de glucides, des fourrages grossiers, des ensilages, des graisses, des vitamines, des minéraux et des oligo-éléments, l'alimentation animale contenant des procyanidines oligomères présentes sous la forme complexée avec des ions ferriques et/ou des composés ferreux, les procyanidines oligomères étant contenues sous la forme d'écorce de pin moulue, d'extrait d'écorce de pin ou d'un mélange quelconque de ces derniers et le rapport des ions ferriques et/ou composés ferreux aux procyanidines oligomères se situant entre 1 : 10 et 1 : 20000, de préférence entre 1 : 100 et 1 : 8000.

3. Alimentation animale destinée à l'utilisation selon la revendication 2, **caractérisée en ce qu'**elle contient de 0,01 g à 250 g d'écorce de pin moulue ayant été traitée avec une solution ferreuse par kilogramme de substance sèche d'alimentation animale.

4. Alimentation animale destinée à l'utilisation selon la revendication 2, **caractérisée en ce qu'**elle contient de 0,002 g à 50 g d'extrait d'écorce de pin ayant été traité avec une solution ferreuse par kilogramme de substance sèche d'alimentation animale.

5. Ecorce de pin moulue, extrait d'écorce de pin ou un mélange quelconque de ces derniers, ayant été traité(e) avec une solution ferreuse, le rapport des ions ferriques et/ou composés ferreux aux procyanidines oligomères contenues dans l'écorce de pin et/ou dans l'extrait d'écorce de pin se situant entre 1 : 10 et 1 : 20000, de préférence entre 1 : 100 et 1 : 8000, les procyanidines oligomères étant présentes sous la forme d'un complexe ferreux, destiné(e) à l'utilisation pour le traitement ou la prophylaxie de l'acidose ruménale aiguë ou subaiguë ou d'états résultant de l'acidose ruménale chez un ruminant.

6. Ecorce de pin moulue, extrait d'écorce de pin ou un mélange quelconque de ces derniers, destiné(e) à l'utilisation selon la revendication 5, le ruminant recevant par jour de 0,1 à 500 g d'écorce de pin moulue, ayant été traitée avec une solution ferreuse.

7. Ecorce de pin moulue, extrait d'écorce de pin ou un mélange quelconque de ces derniers, destiné(e) à l'utilisation selon la revendication 5, le ruminant recevant par jour de 0,002 à 100 g d'extrait d'écorce de pin ayant été traité avec une solution ferreuse.

8. Ecorce de pin moulue, extrait d'écorce de pin ou un mélange quelconque de ces derniers, destiné(e) à l'utilisation selon la revendication 5, l'écorce de pin moulue traitée avec une solution ferreuse, l'extrait d'écorce de pin traité avec une solution ferreuse ou un mélange quelconque de ces derniers se présentant sous la forme d'additif à l'eau potable.

9. Ecorce de pin moulue, extrait d'écorce de pin ou un mélange quelconque de ces derniers, destiné(e) à l'utilisation selon la revendication 8, l'additif à l'eau potable contenant par litre de 0,00008 g à 100 g d'extrait d'écorce de pin ayant été traité avec une solution ferreuse.

10. Ecorce de pin moulue, extrait d'écorce de pin ou un mélange quelconque de ces derniers, destiné(e) à l'utilisation selon l'une quelconque des revendications 5 à 9, les états résultant de l'acidose ruménale chez un ruminant étant choisis dans le groupe comprenant la baisse de pH dans le rumen, la dégradation de la flore ruménique, la suspension de la rumination, la baisse de lactation, la baisse de la teneur en matière grasse laitière, la charge métabolique chronique, la charge métabolique hépatique, la stérilité, la taux de remonte prolongé, la réduction du cheptel des veaux et les affections infectieuses des onglons.

11. Utilisation d'écorce de pin moulue, d'extrait d'écorce de pin ou d'un mélange quelconque de ces derniers, notamment de procyanidines oligomères d'écorce de pin, d'extrait d'écorce de pin ou d'un mélange quelconque de ces derniers ayant été traités avec une solution ferreuse pour la réduction de l'excrétion d'aflatoxine dans le lait d'un ruminant, le rapport des ions ferriques complexables et/ou des composés ferreux complexables aux procyanidines oligomères contenues dans l'écorce de pin et/ou dans l'extrait d'écorce de pin se situant entre 1 : 10 et 1 : 20000, de préférence entre 1 : 100 et 1 : 8000 et les procyanidines oligomères se présentant sous la forme d'un complexe ferreux.
